Europäisches Patentamt

European Patent Office

Office européen des brevets

(19)

(11) Publication number: **0 353 895**
**A1**

## EUROPEAN PATENT APPLICATION

(21) Application number: 89307182.9

(22) Date of filing: 14.07.89

(51) Int. Cl.⁴: **G01N 33/543** , //G01N33/574, **G01N33/78**

(30) Priority: 18.07.88 JP 176962/88

(43) Date of publication of application:
07.02.90 Bulletin 90/06

(84) Designated Contracting States:
CH DE FR GB IT LI NL SE

(71) Applicant: **MITSUI TOATSU CHEMICALS, INCORPORATED**
**2-5, 3-chome, Kasumigaseki**
**Chiyoda-ku Tokyo(JP)**

(72) Inventor: **Tokuda, Chikashi**
**2142 Tougou**
**Mobara-shi Chiba-ken(JP)**
Inventor: **Fukui, Hideo**
**3-9-9 Toubudai**
**Mobara-shi Chiba-ken(JP)**

(74) Representative: **Stuart, Ian Alexander et al**
**MEWBURN ELLIS 2 Cursitor Street**
**London EC4A 1BQ(GB)**

(54) Immunoassay method.

(57) In immunoassays, total measurement time can be greatly reduced without reducing their sensitivities, by replacing a conventional step comprising reacting a substance to be tested with an antibody specific to the substance which antibody is immobilized on a solid phase and detecting the complex consisting of the substance and the antibody formed on the solid phase by a marker. This invention features reacting the substance to be tested with the antibody in a liquid phase, immobilizing the complex on a solid phase and detecting the complex bound to the solid phase by a marker.

The complex formed in the liquid phase can be efficiently immobilized on a solid phase by using a biotinized antibody and an avidin or streptoavidin-bound solid phase.

EP 0 353 895 A1

## IMMUNOASSAY METHOD

The present invention relates to an immunoassay method. A preferred embodiment can provide an improved immunoassay method which permits highly sensitive measurements to be made in a shorter period of time.

The method of the present invention can be used for various testing purposes in fundamental researches and applied fields of medicine and life science, as well as in various test such as clinical examinations, quarantines, diagnostic tests and food inspections.

In highly sensitive immunoassay methods currently in wide use, such as enzyme immunoassays (EIA), radio-immunoassays (RIA) and fluoroimmunoassays (FIA), a reduction in measuring time is very important for the purpose of enhancing the efficiency of researches and routine operations.

Among such immunoassay methods, heterogeneous immunoassays (i.e., immunoassay methods utilizing a heterogeneous system in which an antigen-antibody reaction or a labeling reaction is carried out while one of the reactants is bound to a solid phase) are superior in sensitivity and other characteristics. However, they have the disadvantage of requiring a relatively long period of time for reaction on the solid phase.

On the other hand, homogeneous immunoassays, disclosed in, for example, Ullman, E.F. et al. Homogeneous enzyme immunoassay for thyroxine. Clin. Chem., 21, 1011, 1975, in which the reaction is carried out without immobilizing the reactants are also used popularly in the fields of EIA and FIA, because of their relatively short measuring times. However, they are suited only to the measurement of low-molecular weight haptens and not to the measurement of high-molecular weight substances.

Although homogeneous immunoassays suitable for the measurement of high-molecular weight substances have been put to practical use (Gibbons, I. et al. Homogeneous enzyme immunoassay for proteins employing B-galactosidase. Anal. Biochem., 102, 167, 1980; and Ashihara, Y. et al. Homogeneous enzyme immunoassay for macromolecular antigens using hybrid antibody. J. Clin. Lab. Anal., 1, 77, 1987), all of them are significantly inferior in sensitivity to heterogereous immunoassays. Thus, they cannot be applied to the measurement of haptens and antigens, such as certain hormones and tumor markers, which require very high sensitivity.

**Preferred embodiments of the present invention may** provide an improved immunoassay method which permits highly sensitive measurements to be made in a short period of time.

**Preferred embodiments of the present invention may** provide an improved immunoassay method which permits highly sensitive and time-saving measurements of not only low-molecular-weight substances but also for high-molecular-weight substances.

**The immunoassay method of the present invention generally comprises,** in combination, the steps of, reacting, in a liquid phase, a sample containing a substance to be tested, with an antibody capable of reacting specifically with the substance to be tested and thus serving as a reagent for the detection of the substance to be tested to form a complex; (b) binding the substance to be tested/antibody reagent complex resulting from the reaction, to a solid phase; and (c) detecting the complex by means of a marker.

**In preferred embodiments of the present** invention, the antigen-antibody reaction between the substance to be tested and the antibody reagent is carried out in the liquid phase, so that it proceeds rapidly within a short period of time. As a result, the total measuring time can be greatly reduced because the reaction of the sample with the antibody reagent does not require a long time.

Moreover, in the immunoassay method of the present invention, antigen-antibody reactions (including hapten-antibody reactions) other than that between the substance to be tested and the antibody reagent, such as those between a marker-labeled antibody and the substance to be tested and between a marker-labeled antigen or hapten and the antibody reagent, may also be carried out in the liquid phase. Thus, the measuring time can further be reduced for reasons similar to that described above.

On the other hand, the most time-consuming step, or the rate-determining step, of conventional heterogeneous immunoassays such as EIA, RIA and FIA is the reaction of an antibody bound to a solid phase with an antigen, a hapten or a complex thereof. Accordingly, it is necessary to accelerate this reaction. To this end, a variety of means have been proposed up to now. A typical example is to shake the reaction mixture during the reaction.

However, the rate of an antigen-antibody reaction between solid and liquid phases basically has its limit. Thus, in immunoassays utilizing an antigen-antibody reaction between solid and liquid phases, there has been a limit to the reduction of the total measuring time.

In the immunoassay method of the present invention, the detection of the substance to be tested/antibody reagent complex by means of a marker is carried out either in the absence of unreacted substances or under a reduced concentration of the unreacted substances while the complex is bound to a solid phase, so that its detection by means of a marker can be achieved with high sensitivity.

Thus, the present invention provides an immunoassay method in which full advantage is taken of the high sensitivity of conventional heterogeneous immunoassays and, moreover, the total testing time is greatly reduced.

The present invention also provides an immunoassay method which is useful in time-saving and highly sensitive measurements of not only low-molecular-weight substances but also high-molecular-weight substances.

The immunoassay method of the present invention comprises the steps of (a) reacting a sample containing a substance to be tested, with an immobilizing antibody capable of reacting specifically with the substance to be tested and binding it to a solid carrier, to form a complex of the substance to be tested and the immobilizing antibody; (b) binding the complex formed in step (a) to the solid carrier with the aid of the immobilizing antibody contained in the complex; and (c) subjecting the complex bound to the solid carrier by means of a marker.

One aspect of the present invention is an improvement for conventional immunoassay methods comprising the steps of reacting a sample containing a substance to be tested and an antibody specific to that substance and detecting the resulting complex consisting of the substance and the antibody by a marker to measure the substance in the sample.

This improvement comprising the combination of the steps of reacting the sample and the antibody in a liquid phase and detecting the complex bound to a solid phase by the marker. By this improvement, the total measurement time can be greatly reduced, while the high sensitivity of immunoassays can be maintained. Therefore, this improvement can be applied to any immunoassay using antigen/antibody reaction and detection by a marker.

The substances which can be tested by the immunoassay method of the present invention include tumor markers such as CEA, AFP and CA-50; hormones such as insulin, TSH, HCG, GH, $T_3$, $T_4$ and steroid hormones; and any other antigens and haptens that can be tested by conventional immunoassay methods, irrespective of their molecular weight or constituent components. Among others, the immunoassay method of the present invention is significantly effective for the measurement of trace substances, such as CEA and TSH, which require very high sensitivity.

The markers which can be used in the present invention include marker enzymes for use in various EIAs, such as peroxidase, glucose oxidase, alkaline phosphadase and $\beta$-D-galactosidase; radioisotopes for use in various RIAs, such as $^{125}$I; fluorescent substances such as fluorescein, rhodamine and phycoerythrin; luminescent substances for use in various FIAs, such as luminol, isoluminol and lucigen; and the like.

The immobilizing antibody used in the present invention should be an antibody capable of reacting specifically with the substance to be tested and being bound to a solid carrier. Preferred examples of the immobilizing antibody are biotinized antibodies. Such biotinized antibodies can readily be bound to an avidin-linked solid carrier by means of avidin-biotin combination. Moreover, this avidin-biotin combination can be effected in a short period of time and its binding constant is substantial. For example, when an avidin-biotin combination is compared with the combination of an antigen (or antibody) with an immobilized antibody (or antigen), the binding contant of the former is about 1,000 times as great as that of the latter.

Accordingly, the utilization of avidin-biotin combination makes it possible to bind the complex positively to a solid carrier and thereby perform more sensitive immunoassays in a shorter period of time. Moreover, more satisfactory results can be obtained by using streptoavidin having higher binding power, in place of avidin.

Systems utilizing avidin-biotin combination are being widely used in immunoassays and, in particular, enzyme-linked immunodetections in the field of histochemistry.

In these applications, however, the avidin-biotin combination is utilized only as a means for labeling an antibody with an enzyme, in place of commonly used chemical labeling. The major reasons why avidin-biotin combination is used in these applications are considered to be that the enzyme-labeled antibodies obtained thereby have a lower level of nonspecific adsorption (i.e., a lower background level) than those obtained by chemical combination, and that the combination of biotin or avidin with an enzyme or antibody is technically easier than the direct combination of an enzyme with an antibody.

The utilization of avidin-biotin combination in the present invention departs from the conventional use of avidin-biotin combination and utilizes it for binding of an antigen-antibody reaction product to a solid phase. In the present invention, the use of an immobilizing antibody makes it possible to carry out the reaction of a sample with the immobilizing antibody in a liquid phase.

The solid carrier used in the present invention may be selected from a variety of commonly used solid carriers, according to the method of binding the immobilizing antibody thereto. Where avidin-biotin

3

combination is utilized for that purpose, resins such as polystyrene and nylon; nitrocellulose; and glass can be used.

Biotinization of antibodies can be carried out in the usual manner. The method of the present invention can preferably be applied, for example, to sandwich assays and competition assays.

More specifically, in the case of sandwich assays, an immobilizing antibody is reacted with a sample containing a substance to be tested, and a marker-labeled antibody. In this reaction, the sample and the marker-labeled antibody may be added to the reaction tube at the same time, or one of them may be added thereto shortly after the other. Simultaneous with this reaction, or shortly after that, the reaction product is reacted with a solid carrier having avidin or streptoavidin linked thereto.

As a result of the above-described procedure, a sandwich-like complex in which the substance to be tested is disposed between the immobilizing antibody and the marker-labeled antibody is formed and then bound to the solid carrier. Then, the complex is separated from unreacted marker-labeled antibody. This separation can be carried out, for example, by removing the carrier having the complex bound thereto from the reaction system and rinsing it with a suitable liquid capable of washing off any unreacted marker-labeled antibody.

Thereafter, the marker is detected in a manner suited to the type of marker used. Specifically, the concentration of the substance to be tested can be determined by measuring enzyme activity in EIA, radioactivity in RIA, or fluorescence intensity in FIA.

On the other hand, competition assays are carried out by subjecting an immobilizing antibody to competitive reaction with a substance to be tested, namely an antigen (or hapten) present in a sample, and a marker-labeled antigen (or hapten). After completion of the reaction, or simultaneously with the reaction, the resulting complexes are reacted with avidin or streptoavidin bound to a carrier. After the removal of any unreacted matter, the antigen (or hapten) to be tested can be measured by detecting the marker bound to the carrier in the same manner as described above for sandwich assays. The specific procedures used in these immunoassay methods according to the present invention can be carried out by well-known methods.

The present invention is further illustrated by the following Example and Reference Example. However, these examples are not to be construed to limit the scope of the invention.

The procedures described not in detail in the following Examples were carried out by well-known methods.

Example 1

In this Example 1, carcinoembryonic antigen (CEA) was measured according to the immunoassay method of the present invention.

1) Preparation of standard CEA solutions

CEA (Chemicon Co.) extracted and purified from a metastatic hepatocarcinoma originating from a primary carcinoma of the large intestine was dissolved in a 0.1M phosphate buffer solution (pH 7.3) containing 0.1M NaCl, 0.1% bovine serum albumin and 0.01% $NaN_3$ (hereinafter referred to as buffer solution A). The resulting solution was appropriately diluted to prepare a series of standard CEA solutions.

2) Preparation of a biotin-linked antibody

10 mg of purified mouse monoclonal antibody against CEA (purified anti-CEA MCA) was dissolved in 5 ml of a 0.1M phosphate buffer solution (pH 7.5). After the addition of 5 mg of sulfosuccinimidyl 6-(biotinamido)hexanonate (Pierce Co.), the resulting mixture was incubated at room temperature for 4 hours and then passed through a column of Sephadex G-25. The protein-containing fraction was isolated to obtain a biotin-linked antibody.

Prior to use, this biotin-linked antibody was diluted with buffer solution A to an appropriate concentration.

3) Preparation of avidin-linked polystyrene balls (avidin-PSBs)

4

4 mg of purified avidin (Sigma Co.) was dissolved in 100 ml of a 0.1M phosphate buffer solution (pH 7.3). After the addition of 800 polystyrene balls (PSBs, 6.3 mm in diameter), the resulting mixture was incubated at 37°C for 16 hours. After completion of the incubation, the PSBs were washed with the same buffer solution to obtain avidin-PSBs.

### 4) Preparation of an enzyme-labeled antibody

According to the procedure of S. Yoshitake et al. (Scand. J. Immunol., 10, 81, 1979), β-D-galactosidase derived from Escherichia coli was linked to the Fab' fraction of anti-CEA rabbit antibody. Specifically, anti-CEA rabbit antibody (Daco Co.) was purified to obtain an IgG fraction, which was digested with pepsin to obtain an F(ab')₂ fraction. 3 mg of the F(ab')₂ was dissolved in a 0.1M phosphate buffer solution (pH 6.0). Then, this F(ab')₂ was reduced by adding 2-mercaptoethylamine to the solution so as to give a concentration of 0.01M and incubated at 37°C for 90 minutes. After completion of the reduction, the solution was passed through a column of Sephadex G-25 to obtain an Fab' fraction.

To this Fab' fraction was added 0.5 mg of N,N'-o-phenylene dimaleimide. The resulting mixture was reacted at 30°C for 20 minutes and then passed through a column of Sephadex G-25 to obtain maleimidized Fab'. After its pH was adjusted to 6.5, 9 mg of β-D-galactosidase (Boehringer Mannheim GMbH) was added thereto and the resulting reaction mixture was incubated at 4°C for 24 hours.

This reaction mixture was purified by passage through a column of Sepharose 6B. Thus, there was obtained an enzyme-labeled antibody (i.e., β-D-galactosidase-labeled Fab'). Prior to use, this enzyme-labeled antibody was diluted with buffer solution A to an appropriate concentration.

### 5) Measurement of CEA

To glass test tubes (10 mm in diameter) were added 50 μl of a standard CEA solution or a serum sample, 50 μl of an enzyme-labeled antibody solution, and 150 μl of a biotin-linked antibody solution. These test tubes were incubated at 37°C for 20 minutes. After the addition of one avidin-PSB, the incubation was continued for an additional 8 minutes. Then, the PSB was washed by adding 2 ml of physiological saline and sucking it off, and this procedure was repeated three times.

After the final washing fluid was removed, 0.3 ml of a 0.1M phosphate buffer solution (pH 7.0) containing 0.1mM 4-methylumbelliferyl-β-D-galactosidase as a substrate was added to each test tube and incubated 37°C for 5 minutes. Then, the reaction was stopped by the addition of 1 ml of a 0.1M glycine-NaOH buffer solution (pH 10.3) and the reaction mixture was subjected to fluorometry at an excitation wavelength of 360 nm and an emission wavelength of 420 nm. The results thus obtained are shown in Table 1.

### Reference Example 1

In this Reference Example 1, CEA was measured according to a conventional technique.

### 1) Preparation of an antibody-PSB

The same purified anti-CEA MCA as used in Section 2) of Example 1 was dissolved in a 0.1M phosphate buffer solution (pH 7.3). The concentration of this solution was so adjusted as to give an absorbance of 0.05 at 280 nm. 800 PSBs were added to 100 ml of the above solution and this mixture was incubated at 37°C for 16 hours. Thereafter, the PSBs were washed with the same buffer solution to obtain antibody-PSBs.

### 2) Other reagents

The enzyme-labeled antibody and the standard CEA solutions were the same as those used in Example 1.

3) Measurement of CEA

To test tubes were added 50 μl of a standard CEA solution or a serum sample, 50 μl of an enzyme-labeled antibody solution, 150 μl of a buffer solution and one antibody-PSB. These test tubes were incubated at 37°C for 2 hours. Thereafter, they were subjected to washing, enzyme reaction and fluorometry in the same manner as described in Example 1. The results thus obtained are also shown in Table 1.

Table 1

| Standard Curves for CEA in Example 1 and Reference Example 1 | | | | | |
|---|---|---|---|---|---|
| (Fluorescence intensity; average of duplicate measurements) | | | | | |
| | CEA concentration (ng/ml) | | | | |
| | 0 | 5 | 10 | 30 | 60 |
| Example 1 | 12.5 | 38.1 | 56.0 | 146 | 258 |
| Reference Example 1 | 10.4 | 30.2 | 58.8 | 156 | 264 |

As is evident from the results shown in Table 1, the immunoassay method of the present invention reduced the antigen-antibody reaction time from 2 hours (according to the conventional technique) to 10 minutes while maintaining the measuring sensitivity at substantially the same level.

Example 2

In this example, blood 3,3',5-L-triiodothyronine ($T_3$) was measured by the immunoassay method of the present invention.

1) Preparation of standard $T_3$ solutions

$T_3$ (Sigma) was dissolved in a 0.05 N sodium hydroxide solution and the resulting solution was appropriately diluted to prepare a series of standard $T_3$ solutions.

2) Preparation of a biotin-linked antibody

10 mg of purified anti-$T_3$ rabbit antibody was dissolved in 5 ml of a 0.1M phosphate buffer solution (pH 7.5). After addition of 5 mg sulfosuccinimidyl 6-(biotinamido)hexanonate (Pierce Co.), the resulting mixture was incubated at room temperature for 4 hours and then passed through a column of Sephadex G-25. The protein-containing fraction was separated to obtain a biotin-linked antibody.

Prior to use, this biotin-linked antibody was diluted with buffer solution B [0.1M barbital buffer solution (pH 8.6) containing 0.1% bovine serum albumin, 0.01% 8-anilino-1-raphtalene sulfonic acid and 0.01% $NaN_3$] to an appropriate concentration.

3) Preparation of an enzyme-labeled hapten

4 mg of $T_3$ was dissolved in 2 ml of dimethylformamide. After addition of 0.8 mg of succinimidyl 4-(N-maleimidomethyl)cyclohexane-1-carboxylate, the resulting mixture was incubated at room temperature for 90 minutes. After addition of 0.1 ml of 1M glycine to the resulting mixture. 6 mg of $\beta$-D-galactosidase derived from E. coli (Boeringer Mannheim GmbH) dissolved in 4.0 ml of a 0.1M phosphate buffer solution (pH 7.3) was added. Then the mixture was further incubated at 30°C for 30 minutes.

After the incubation, the reaction mixture was passed through a column of Sephadex G-25. The protein-containing fraction was separated to obtain an enzyme-labeled hapten (β-D-galactosidase-labeled T₃).

Prior to use, this enzyme-linked hapten was diluted with buffer solution B to an appropriate concentration.

4) Measurement of T₃

To glass test tubes (10 mm in diameter) were added 50 μl of either a standard T₃ solution or a serum sample; 100 μl of an enzyme-labeled hapten solution and 150 μl of a biotin-linked antibody solution. These test tubes were incubated at 37°C for 2 minutes. After addition of one avidin-PSB prepared by the procedure of Example 1, Section 3, the incubation was continued for an additional 8 minutes. Then, the PBS was washed by adding 2 ml of physiological saline and sucking it off, and this procedure was repeated three times.

After the final washing fluid was removed, 0.3 ml of a 0.1M phosphate buffer solution (pH 7.0) containing 0.1 mM 4-methylumbelliferyl-β-D-galactosidase as a substrate was added to each test tube and incubated at 37°C for 5 minutes. Then, the reaction was stopped by addition of 1.0 ml of a 0.1M glycine-NaOH buffer solution (pH 10.3) and the reaction mixture was subjected to fluorometry at an excitation wavelength of 360 nm and an emission wavelength of 420 nm. The results thus obtained are shown in Table 2.

Reference Example 2

In this reference example 2, T₃ was measured according to a conventional technique.

1) Preparation of an antibody-PSB

The same purified anti-T₃ antibody as used in Example 2, Section 2), was dissolved in a 0.1M phosphate buffer solution (pH 7.3). The concentration of this solution was so adjusted as to give an absorbance of 0.002 at 280 nm. In the same manner as described in Reference Example 1, Section 3), 800 PSBs were added to 100 ml of this solution and the resulting mixture was incubated at 37°C for 16 hours. Thereafter, the PSBs were washed with the same buffer solution to obtain antibody PSBs.

2) Other reagents

The enzyme-labeled hapten and the T₃ standard solutions were the same as those used in Example 2.

3) Measurement of T₃

To test tubes were added 50 μl of either a standard T₃ solution or a serum sample; 100 μl of a enzyme-labeled hapten solution; 150 μl of buffer solution B; and one antibody-PSB. These test tubes were incubated at 37°C for 2 hours. Thereafter, they were subjected to washing, enzyme reaction and fluorometry in the same manner as described in Example 2, Section 4). The results thus obtained are also shown in Table 2.

EP 0 353 895 A1

Table 2

| Standard Curves for $T_3$ in Example 2 and Reference Example 2 | | | | | |
|---|---|---|---|---|---|
| (Fluorescence intensity; average of duplicate measurements) | | | | | |
| | $T_3$ Concentration (ng/ml) | | | | |
| | 0 | 0.75 | 1.5 | 3.0 | 6.0 |
| Example 2 | 278 | 196 | 148 | 92.2 | 45.3 |
| Reference Example 2 | 295 | 191 | 132 | 94.4 | 46.5 |

## Claims

1. An immunoassay method comprising the steps of

(a) reacting a sample containing a substance to be tested, with an immobilizing antibody capable of reacting specifically with the substance to be tested and binding it to a solid carrier, to form a complex of the substance to be tested and the immobilizing antibody;

(b) binding the complex formed in step (a) to the solid carrier with the aid of the immobilizing antibody contained in the complex; and

(c) detecting the complex bound to the solid carrier by means of a marker.

2. An immunoassay method comprising the steps of

(a) reacting a sample containing a substance to be tested with an immobilizing antibody and a marker-labelled antibody specific for the substance to be tested;

(b) binding the immobilizing antibody/substance to be tested/marker-labelled antibody complex formed in step (a) to the solid carrier to separate the complex from unreacted marker-labelled antibody; and

(c) detecting the complex bound to the solid carrier by means of the marker contained in the complex.

3. An immunoassay method as claimed in claim 2, wherein, in step (a), the complex is formed by reacting the sample with the immobilizing antibody and then reacting the resultant reaction mixture with the marker-labelled antibody.

4. An immunoassay method as claimed in claim 2, wherein, in step (a), the complex is formed by reacting the same with the marker-labelled antibody and then reacting the resultant reaction mixture with the immobilizing antibody.

5. An immunoassay method as claimed in claim 2, wherein, in step (a), the complex is formed by reacting the sample with both of the marker-labelled antibody and the immobilizing antibody at the same time.

6. An immunoassay method comprising the steps of

(a) subjecting an immobilizing antibody to competitive reaction with a sample containing a substance to be tested and either a marker-labelled antigen or hapten, said antigen and hapten being specific for the immobilizing antibody;

(b) binding the complexes formed in step (a) to the solid carrier to separate the complexes from unreacted marker-labelled antigen or hapten; and

(c) detecting the immobilizing antibody/marker-labelled antigen or hapten complex bound to the solid carrier by means of the marker.

7. An immunoassay method as claimed in any preceding claim wherein the substance to be tested is an antigen or a hapten.

8. An immunoassay method as claimed in any preceding claim wherein the immobilizing antibody is a biotinized antibody capable of being bound to the solid carrier by combination with avidin linked to the solid carrier.

9. An immunoassay method as claimed in any preceding claim wherein the marker is an enzyme, a fluorescent substance, a luminescent substance or a radioisotope.

8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 201 079 (R. ZAHRADNIK)<br>* Page 5, line 7 - page 6, line 26; claim 1 *<br>--- | 1-9 | G 01 N 33/543//<br>G 01 N 33/574<br>G 01 N 33/78 |
| X | WO-A-8 404 171 (QUIDEL)<br>* Claim 2 *<br>--- | 1-9 | |
| X | PATENT ABSTRACTS OF JAPAN, vol. 8, no. 144 (P-284)[1581], 5th July 1984, page 105 P 284; & JP-A-59 43 360 (OLYMPUS KOGAKU KOGYO K.K.) 10-03-1984<br>* Abstract *<br>--- | 1-9 | |
| Y | DE-A-2 951 678 (E.Y. LAB. INC.)<br>* Abstract; page 12, line 13 - page 15, line 16; claims *<br>--- | 1-9 | |
| Y | US-A-4 343 896 (G. WOLTERS et al.)<br>* Abstract; column 1, lines 49-66; column 2, line 60 - column 3, line 2; column 3, line 60 - column 4, line 11; claims 1-6 *<br>--- | 1-9 | TECHNICAL FIELDS SEARCHED (Int. Cl.5) |
| Y | US-A-4 253 995 (D.H. KATZ)<br>* Abstract; column 3, lines 23-29; column 14, line 61 - column 15, line 8; claim 1 *<br>--- | 1-9 | G 01 N |
| A | US-A-4 228 237 (R.C. HEVEY et al.)<br>* Abstract; column 1, line 1 - column 3, line 21; claims 1-4,6-10,13 *<br>--- | 1-9 | |
| A | EP-A-0 160 900 (ABBOTT LAB.)<br>* Abstract; page 3, line 13 - page 5, line 4; claims 1-7 *<br>----- | 1-9 | |

The present search report has been drawn up for all claims

| Place of search | Date of completion of the search | Examiner |
|---|---|---|
| THE HAGUE | 04-10-1989 | DIEDENHOFEN A. |